# FASCICULE DE BREVET EUROPEEN

(11) **EP 1 211 258 B1**
(45) Date de publication et mention de la délivrance du brevet: **29.09.2004**
(21) Numéro de dépôt: 01402808.8
(22) Date de dépôt: 30.10.2001
(51) Int. Cl.: C07H 3/06, C07H 15/04, C12P 19/44, A61K 7/48, A61K 7/50, A61K 7/06, A61K 47/36, A01N 25/30, A61K 7/16, B01F 17/42, B01F 17/56, B01F 17/00, C11D 1/66

(54) **Procédé de préparation d'adjuvants de solubilisation à partir d'huiles de fusel et d'oses**
Verfahren zur Herstellung von Solubilisierzusatzstoffen aus Fuselölen und Osen
Process for preparing solubilizing adjuvants from fusel oils and oses

(30) Priorité: 14.11.2000 FR 0014589
(43) Date de publication de la demande: 05.06.2002
(73) Titulaire: AGRO INDUSTRIE RECHERCHES ET DEVELOPPEMENTS (A.R.D.), 51110 Pomacle (FR)
(72) Inventeur: Bertho, Jean-Noel, 08300 Neuflize (FR); de Baynast, Régis, 78000 Versailles (FR)
(74) Mandataire: Eidelsberg, Victor Albert

(56) Documents cités:
- EP-A- 0 062 027
- EP-A- 0 699 472
- EP-A- 0 895 805
- EP-A- 1 027 921
- FR-A- 2 744 648
- US-A- 3 450 690
- PATENT ABSTRACTS OF JAPAN vol. 1998, no. 04, 31 mars 1998 (1998-03-31) & JP 09 315932 A (SHISEIDO CO LTD), 9 décembre 1997 (1997-12-09)

## Description

La présente invention se rapporte aux procédés de fabrication d'adjuvants de solubilisation, à leurs applications et à leurs formulations.

Au FR-2 744 648, au EP-A-0895 805 et au EP-A-1 027 921 on prépare des alkyl glycosides à partir de bagasse, de matière première d'origine végétale ou de sirops provenant de matière riches en amidon et en hémicellulose.

Les huiles de Fusel forment des liquides incolores ou jaunâtres, qui possèdent une odeur caractéristique. Elles présentent une densité d'environ 0,83. Leur point d'ébullition est loin d'être constant, car elles constituent des mélanges complexes de corps à point d'ébullition très variable : l'ébullition commence à environ 80°C et s'élève jusque à 130-134°C.

Les huiles de Fusel sont des coproduits fatals de la fermentation alcoolique. Produites par la levure en anaérobiose à partir des matières azotées, elles sont récupérées après rectification où sur les plateaux de milieu de colonne pour lesquels les liquides alcooliques titrent 40-50°. Insolubles dans l'eau, elles sont le plus souvent lavées à l'eau et décantées afin de réduire d'environ 4% à 5% la quantité d'éthanol qu'elles contiennent. Dans la présente invention, tous les pourcentages sont exprimés en poids.

Les huiles de Fusel représentent en moyenne 2 à 5 ‰ due l'alcool éthylique fabriqué. La production d'alcool éthylique en France étant de 3 millions d'hectolitres pour l'industrie, sans compter les biocarburants, le gisement potentiel est donc d'environ 900 tonnes.

Les huiles de Fusel dites parfois « huiles amyliques » ou « Fusels », ont des compositions qui varient en fonction de leurs origines (moûts de pommes de terre, de betteraves, de blé, d'orge...).
Il s'agit d'un mélange :
- De 5 à 20% d'eau,
- De 60 à 95% d'alcools majoritairement constitués d'alcanols linéaires ou ramifiés présentant de 2 à 5 atomes de carbone,
- d'impuretés (furfurols, éthers, acides gras...) qui peuvent aller dans les cas extrêmes jusqu'à 15%.

La répartition des principaux alcanols est la suivante :

| **Alcanol** | **Teneur (%)** | **Formule** |
|---|---|---|
| Ethanol | 5 à 40 | CH₃-CH₂-OH |
| 1-Propanol | 1 à 8 | CH₃-(CH₂)₂-OH |
| 2-propanol | 0 à 1 | CH₃-CH(OH)-CH₃ |
| 2-Méthyl-propanol | 5 à 15 | CH₃-CH(CH₃)-CH₂-OH |
| 1-Butanol | 0 à 1 | CH₃-(CH₂)₃-OH |
| 2-Méthyl-butanol | 10 à 30 | CH₃-CH₂-CH(CH₃)-CH₂-OH |
| 3-Méthyl-butanol | 25 à 70 | CH₃-CH(CH₃)-(CH₂)₂-OH |

L'ensemble des alcanols représentant 100%.

Notons que les alcools de Fusel sont des alcools naturels directement produits par voie biotechnologique au niveau des distilleries, sans aucune étape chimique intermédiaire.

L'invention a pour objet un procédé de préparation d'adjuvant de solubilisation caractérisé en ce qu'il consiste à mettre en contact des huiles de Fusel avec un ou plusieurs sucres réducteurs en présence d'un catalyseur acide, à une température comprise entre 50 et 130°C et en éliminant l'eau du milieu réactionnel jusqu'à obtention d'une solution de glycosides d'alkyle et à séparer les glycosides de cette solution.

Par sucre réducteur, on entend des oses réducteurs choisis parmi les aldoses tels que le thréose, l'érythrose, le xylose, le lyxose, le ribose, l'arabinose, le glucose, le galactose, le mannose, l'idose, le gulose, le talose, l'allose ou l'altrose ; les cétoses tels que le fructose, le sorbose, l'érythrulose... ; les disaccharides tels que le maltose, les oligosaccharides et polysaccharides tels que l'amidon, les dextranes, les arabino-xylanes, les pentosanes , les xylanes. On entend également par sucre réducteur, les acides uroniques tels que l'acide galacturonique, l'acide glucuronique, l'acide mannuronique. On entend de plus comme sucre réducteur les disaccharides et oligosaccharides non réducteurs comme par exemple le saccharose qui, en présence de catalyseur acide tel que l'acide sulfurique conduisent à des monosaccharides réducteurs. On entend enfin comme sucre réducteur des mélanges de ces sucres précédents.

Chaque ose peut être sous forme isomérique α ou β, sous forme L ou D, et sous forme furanosique ou pyranosique.

On préfère les pentoses et tout particulièrement le L-arabinose et le D-xylose qui sont abondamment présents dans les hémicelluloses de nombreux végétaux.

On utilise également les hexoses de la série D, notamment le D-glucose en raison de son abondance sur le marché des sucres.

On apprécie tout particulièrement les mélanges de sucres réducteurs majoritairement constitués de D-glucose et de pentoses, notamment de D-Xylose et de L-Arabinose. De préférence on préfère utiliser des mélanges de sucres réducteurs dérivés de coproduits agricoles riches en hémicellulose et/ou en amidon comme par exemple la paille de blé, le son brut ou désamylacé de blé, les coproduits d'amidonnerie tels que définis dans le brevet EP 0 699 472, les coproduits agricoles tels que définis dans le brevet EP 0 880 538 et plus particulièrement les mélanges de sucres réducteurs contenant de 25 à 98%, de préférence 60 à 100% et plus particulièrement 90 à 100% de pentoses et 0 à 34%, et 2 à 75%, de préférence 0 à 40% et plus particulièrement 0 à 10% d'hexoses.

Les sucres ou les mélanges de sucres réducteurs peuvent être cristallisés ou de préférence utilisés sous forme de sirops.

Le premier stade du procédé suivant l'invention appelé communément glycosylation, consiste à mettre en contact les huiles de Fusel avec des sucres en présence d'un catalyseur acide et en éliminant l'eau du milieu réactionnel. On préfère cependant avant la mise en contact, purifier les huiles de Fusel. Cette étape est avantageusement réalisée par rectification. Elle permet l'élimination des résidus lourds des huiles de Fusel ( majoritairement constitués des impuretés) qui présentent des points d'ébullition supérieurs à 140°C. Elle permet également d'éliminer en plus des fractions lourdes les fractions légères qui présentent des points d'ébullition inférieurs à 100°C et qui sont majoritairement constituées d'eau, d'éthanol, de propanol et de 2-méthylpropanol. On utilise en général 1 à 20 équivalents molaires d'alcanols par rapport aux sucres et de préférence 1,5 à 6 équivalents.

Pendant la mise en contact, on greffe les alcanols contenus dans les huiles de Fusel brutes ou purifiées sur les carbones anomériques des sucres pour fabriquer les glycosides d'alkyle.

La mise en contact est réalisée en présence d'un catalyseur acide tel que l'acide sulfurique, un acide sulfonique tel que l'acide méthanesulfonique, l'acide chlorhydrique, l'acide hypophosphoreux ou tout autre catalyseur acide permettant d'effectuer une glycosidation et leurs mélanges. Cette catalyse acide peut être également effectuée par 0,05 à 6 équivalents pondéraux d'une résine sulfonique sous sa forme H⁺, ou d'une résine acide.

La mise en contact est effectuée à une température comprise entre 50 et 130°C et de préférence entre 90 et 110°C pendant une durée de 15 minutes à 3 heures et de préférence de 1 heure à 2 heures.

Afin d'obtenir des rendements quantitatifs, il est préférable d'éliminer au cours de la glycosylation, l'eau présente initialement dans les matières premières ou libérée pendant la réaction. Cette opération est avantageusement réalisée par distillation azéotropique au moyen d'une colonne de rectification. Les vapeurs condensées sont décantées dans un décanteur statique ; la phase inférieure riche en eau est éliminée et la phase supérieure assure le reflux de la colonne de rectification.

On préfère effectuer cette réaction de glycosylation en l'absence totale de solvants, mais on peut le cas échéant utiliser un solvant tel qu'un éther oxyde tel que le tétrahydrofurane, l'oxyde diéthylique, le 1,4-dioxane, l'oxyde isopropylique, le méthyltertiobutyl éther, l'éthyl-tertiobutyl éther ou le diglyme, un hydrocarbure halogéné tel que le chlorure de méthylène, le chloroforme, le 1,2-dichloroéthane, un solvant nitré tel que le nitrométhane, le nitro-2-propane, un solvant de la famille des amides tel que le N-méthylformamide, le N,N-diméthylformamide, le N,N-diméthylacétamide, la N-méthyl-2-pyrrolidone, un nitrile tel que l'acétonitrile, un alcane comme l'hexane, l'heptane, ou l'octane, ou un solvant aromatique tel que le toluène ou le xylène.

La mise en contact peut également être réalisée en présence de micro-ondes.

Pour recueillir le mélange de glycosides d'alkyle, le procédé consiste :
- à éliminer le solvant de réaction, s'il est présent,
- à neutraliser le catalyseur acide, puis à filtrer le sel obtenu. On effectue la neutralisation, par exemple, par un hydrogénocarbonate ou un carbonate de métal alcalin ou alcalino-terreux, notamment l'hydrogénocarbonate de sodium, par un hydroxyde de métal alcalin ou alcalino-terreux, notamment la soude, ou par une base organique telle que la triéthanolamine,
- à purifier le produit souhaité :
   - soit par évaporation des alcanols en excès sous vide compris entre 0,001 et 100 mbars à une température comprise entre 60 et 200°C, de préférence au moyen d'un évaporateur couche mince à une pression de 1 à 50 mb et une température de 60 à 120°C,
   - soit par chromatographie sur colonne de gel de silice, d'alumine, de charbon actif ou sur résine échangeuse d'ions,
   - soit, si les produits synthétisés le permettent, par cristallisation dans un solvant ou un mélange de solvants appropriés choisis parmi les éthers oxydes tel que le tétrahydrofurane, l'oxyde diéthylique, le 1,4-dioxane, l'oxyde isopropylique, le méthyltertiobutyl éther, l'éthyl-tertiobutyl éther ou le diglyme, un hydrocarbure halogéné tel que le chlorure de méthylène, le chloroforme, le 1,2-dichloroéthane, un solvant nitré tel que le nitrométhane, le nitro-2- propane, un solvant de la famille des amides tel que le N-méthylformamide, le N,N-diméthylformamide, le N,N-diméthylacétamide, la N-méthyl-2-pyrrolidone, un nitrile tel que l'acétonitrile, un alcane comme l'hexane, l'heptane, ou l'octane, ou un solvant aromatique tel que le toluène ou le xylène,
   - soit par extractions sélectives par des solvants non miscibles à l'eau,

Le produit isolé présente alors un pourcentage d'alcanols issus de l'huile de Fusel résiduel compris entre 0 et 5% et de préférence entre 0 et 1%.
- éventuellement solubiliser le glycoside d'alkyle dans l'eau pour obtenir un adjuvant de solubilisation présentant préférentiellement une matière sèche de 40 à 80 %,
- si besoin, décolorer cet adjuvant en ajoutant à une température comprise entre 15 et 100 °C, 0,05 à 10 % et de préférence de 0,5 à 3 % de peroxyde d'hydrogène, de peroxodisulfates de métaux alcalins ou alcalino-terreux, de perborates, de persulfates, de perphosphates, de percarbonates, d'ozone ou encore de periodinates. On préfère le peroxyde d'hydrogène à 30 ou 50 %. L'agent de décoloration de la présente invention doit naturellement être compatible avec l'ensemble des ingrédients de la formulation finale et avec les applications des produits finis.

La présente invention a également pour objet des adjuvants comprenant en poids à l'exception des impuretés :
- de 0% à 20 % d'un mélange de glycosides de formule (I):

   H₃C-CH₂-O(G₁)ₐ(G₂)_{b}(G₃)_{c}(G₄)_{d}(G₅)ₑ (I)
- de 0% à 5% d'un mélange de glycosides de formule (II):

   H₃C-CH₂-CH₂-O(G₁)ₐ(G₂)_{b}(G₃)_{c}(G₄)_{d}(G₅)ₑ (II)
- de 0% à 15% d'un mélange de glycosides de formule (III): 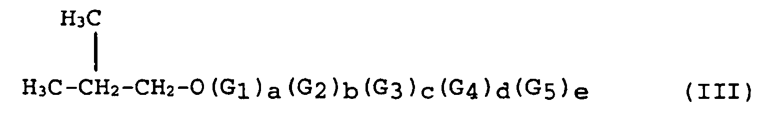
- de 20% à 80% d'un mélange de glycosides de formule (IV): 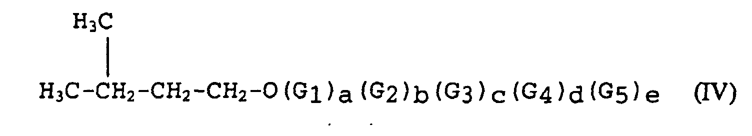
- de 10% à 40% d'un mélange de glycosides de formule (V): 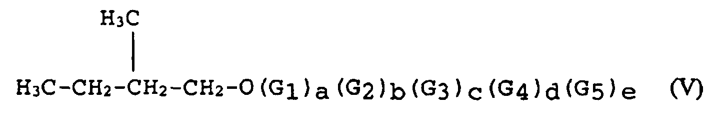
dans lesquelles G₁, G₂, G₃, G₄, G₅ sont indépendamment les uns des autres, des restes d'un ose choisi préférentiellement parmi les hexoses et les pentoses, ces derniers étant choisis préférentiellement parmi l'arabinose et le xylose ; a, b, c, d et e étant égaux à 0 ou à 1, la somme de a, b, c, d et e étant au moins égale à 1. L'ensemble des composés I, II, III, IV et V, hormis les impuretés et d'éventuels glycosides d'alkyle autres que les composés I, II, III, IV et V, représentent 100%.

En raison de leur efficacité et de leur facilité de préparation, on préfère les adjuvants qui comprennent au moins en poids :
- de 0% à 20% d'un mélange de polyglycosides de formule (III),
- de 45% à 80% d'un mélange de polyglycosides de formule (IV),
- de 10% à 40% d'un mélange de polyglycosides de formule (V),
et plus particulièrement les adjuvant qui comprennent en poids :
- de 60% à 75% d'un mélange de polyglycosides de formule (IV),
- de 25% à 40% d'un mélange de polyglycosides de formule (V) .

En pratique, il existe trois principales voies pour obtenir les adjuvants selon l'invention à partir de sucres réducteurs et d'huile de Fusel.

La première voie consiste à mettre en contact séparément des huiles de Fusel avec un sucre réducteur en présence d'un catalyseur acide, à une température comprise entre 50 et 130°C et en éliminant l'eau du milieu réactionnel jusqu'à obtention d'une solution de glycosides d'alkyle et à séparer les glycosides de cette solution. Ensuite, les glycosides d'alkyle fabriqués à partir de différents sucres réducteurs sont éventuellement mélangés afin d'obtenir les adjuvants suivant l'invention.

La seconde voie consiste à mélanger différents sucres réducteurs et à mettre en contact ces mélanges de sucres réducteurs avec des huiles de Fusel, en présence d'un catalyseur acide, à une température comprise entre 50 et 130°C et en éliminant l'eau du milieu réactionnel jusqu'à obtention d'une solution de glycosides d'alkyle et à séparer les glycosides de cette solution afin d'obtenir les adjuvants suivant l'invention.

Enfin, la troisième voie consiste à utiliser des sirops de mélanges de sucres réducteurs dérivés de matières premières végétales riches en amidon et en hémicellulose contenant de 25 à 98%, de préférence 60 à 100% et plus particulièrement 90 à 100% de pentoses et 0 à 34%, et 2 à 75%, de préférence 0 à 40% et plus particulièrement 0 à 10% d'hexoses et d'effectuer la mise en contact de ces sirops de sucres réducteurs avec des huiles de Fusel, en présence d'un catalyseur acide, à une température comprise entre 50 et 130°C et en éliminant l'eau du milieu réactionnel jusqu'à obtention d'une solution de glycosides d'alkyle et à séparer les glycosides de cette solution afin d'obtenir les adjuvants suivant l'invention.

La présente invention a également pour objet une composition comprenant en poids :
- 10 à 60% d'adjuvant ou mélange d'adjuvants suivant l'invention,
- 40 à 90% de tensioactifs non ioniques, anioniques, amphotères, cationiques ou leurs mélanges.

Les tensioactifs anioniques peuvent être :
- des alkylesters sulfonates de formule R-CH(SO₃M)-COOR', où R représente un radical alkyle en C₈₋₂₀, de préférence en C₁₀-C₁₆, R' un radical alkyle en C₁-C₆, de préférence en C₁-C₃ et M un cation alcalin (sodium, potassium, lithium), ammonium substitué ou non substitué (méthyl-, diméthyl-, triméthyl-, tétraméthylammonium, diméthylpiperidinium...) ou dérivé d'une alcanolamine (monoéthanolamine, diéthanolamine, triéthanolamine...);
- des alkylsulfates de formule ROSO₃M, où R représente un radical alkyle ou hydroxyalkyle en C₁₀-C₂₄, de préférence en C₁₂-C₂₀ et tout particulièrement en C₁₂-C₁₈, M représentant un atome d'hydrogène ou un cation de même définition que ci-dessus, ainsi que leurs dérivés éthoxylénés (OE) et/ou propoxylénés (OP), présentant en moyenne de 0,5 à 6 motifs, de préférence de 0,5 à 3 motifs OE et/ou OP ;
- les alkylamides sulfates de formule RCONHR'OSO₃M où R représente un radical alkyle en C2-C22, de préférence en C6-C20, R' un radical alkyle en C2-C3, M représentant un atome d'hydrogène ou un cation de même définition que ci-dessus, ainsi que leurs dérivés éthoxylénés (OE) et/ou propoxylénés (OP), présentant en moyenne de 0,5 à 60 motifs OE et/ou OP ;
- les alkyl-D-galactosides uronates de formule VI

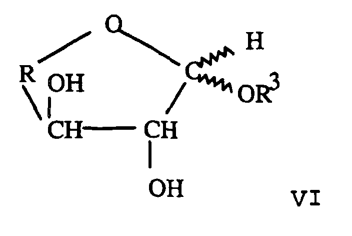

R³ étant un radical linéaire ou ramifié alkyle de 6 à 22 atomes de carbone et de préférence de 8 à 16 atomes de carbone, hydrocarboné ayant de 1 à 4 insaturations éthyléniques et de 6 à 22 atomes de carbone ou l'un de ces radicaux substitués par 1 à 3 substituants sur des atomes de carbone différents choisis parmi hydroxy, halogène et trifluorométhyle, R étant

〉CH-CH(OH)-CO₂R⁴

ou 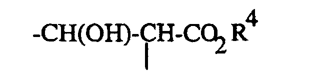 dont le carbone portant le groupe hydroxy n'est pas relié à l'atome d'oxygène endocyclique. R⁴ étant l'hydrogène, un atome de métal alcalin, de métal alcalino-terreux, un groupe ammonium quaternaire ;
- les sels d'acides gras saturés ou insaturés en C₈-C₂₄, de préférence en C₁₄-C₂₀, les alkylbenzènesulfonates en C₉-C₂₀, les alkylsulfonates primaires ou secondaires en C₈-C₂₂, les alkylglycérol sulfonates, les acides polycarboxyliques sulfonés décrits dans GB-A-1 082 179, les sulfonates de paraffine, les N-acyl N-alkyltaurates, les alkylphosphates, les alkyliséthionates, les alkylsuccinamates, les alkylsulfosuccinates, les monoesters ou diesters de sulfosuccinates, les N-acyl sarcosinates, les sulfates d'alkylglycosides, les polyéthoxycarboxylates, le cation étant un métal alcalin (sodium, potassium, lithium), un reste ammonium substitué ou non substitué (méthyl-, diméthyl-, triméthyl-, tétraméthylammonium, diméthylpiperidinium...) ou dérivé d'une alcanolamine (monoéthanolamine, diéthanolamine, triéthanolamine.

Les tensioactifs non ioniques peuvent être :
- les alkylphénols polyoxyalkylénés (polyéthoxyéthylénés, polyoxypropylénés, polyoxybutylénés) dont le substituant alkyle est en C₆-C₁₂ et contenant de 5 à 25 motifs oxyalkylènes ; à titre d'exemple, on peut citer les TRITON X-45, X-114, X-100 ou X-102 commercialisés par Rohm & Haas Cy.;
- les glucosamides, glucamides ;
- les glycérolamides dérivés de N-alkylamines (US-A-5,223,179 et FR-A-1,585,966) ;
- les alcools aliphatiques en C₈-C₂₂ polyoxyalkylénés contenant de 1 à 25 motifs oxyalkylènes (oxyéthylène, oxypropylène) ; à titre d'exemple, on peut citer les TERGITOL 15-S-9, TERGITOL 24-L-6 NMW commercialisés par Union Carbide Corp., NEODOL 45-9, NEODOL 23-65, NEODOL 45-7, NEODOL 45-4 commercialisés par Shell Chemical Cy., KYRO EOB commercialisé par The Procter & Gamble Cy ;
- les produits résultant de la condensation de l'oxyde d'éthylène avec un composé hydrophobe résultant de la condensation de l'oxyde de propylène avec le propylène glycol, tels les PLURONIC commercialisés par BASF ;
- les produits résultant de la condensation de l'oxyde d'éthylène le composé résultant de la condensation de l'oxyde de propylène avec l'éthylènediamine, tels les TETRONIC commercialisés par BASF ;
- les oxydes d'amines tels que les oxydes d'alkyl C₁₀-C₁₈ diméthylamimes, les oxydes d'alkoxy C8-C22 éthyl dihydroxy éthylamines ;
- les alkylpolyglycosides de formule VII suivante :

   R⁵-O(A₁)_{f}(A₂)_{g}(A₃)ₕ(A₄)ᵢ(A₅)ⱼ (VII)
dans lesquelles A₁, A₂, A₃, A₄, A₅ sont indépendamment les uns des autres, des restes d'un ose choisi parmi les hexoses et plus particulièrement le D-glucose ; les pentoses, ces derniers étant choisis préférentiellement parmi l'arabinose et le xylose ; f, g, h, i et j étant égaux à 0 ou à 1, la somme de f, g, h, i et j étant au moins égale à 1 ; R⁵ étant un radical linéaire ou ramifié alkyle de 6 à 22 atomes de carbone, hydrocarboné ayant de 1 à 4 insaturations éthyléniques et de 6 à 22 atomes de carbone ou l'un de ces radicaux substitués par 1 à 3 substituants sur des atomes de carbone différents choisis parmi hydroxy, halogène et trifluorométhyle ;
- les amides d'acides gras en C8-C20 ;
- les acides gras éthoxylés ;
- les amides gras éthoxylés ;
- les amines éthoxylées.

Les tensioactifs cationiques peuvent être par exemple des halogénures d'alkyldiméthylammonium.

Les agents tensioactifs amphotères et zwitterioniques peuvent être :
- les alkyldiméthylbétaïnes,
- les alkylamidopropyldiméthylbétaïnes,
- les alkyltriméthylsulfobétaïnes,
- les produits de condensation d'acides gras et d'hydrolysats de protéines.

Les compositions selon l'invention permettent d'augmenter la solubilité de molécules lipophiles dans l'eau. Les molécules lipophiles peuvent être :
- des huiles essentielles telles que par exemple, les huiles de pin maritime ou sylvestre, les huiles d'agrumes tels que le citron, le pamplemousse, l'orange, la mandarine, les huiles de céréales telles que l'huile de gluten de blé, l'huile de germe de blé, les huiles d'anis, d'amande amère, de bouleau, de camomille, de bergamote, de cannelle, de citronnelle, de plantes aromatiques telles que le thym blanc, le thym rouge, le romarin, la menthe, l'eucalyptus, le basilic, l'estragon, le laurier, l'origan, la verveine, les huiles de genièvre, de girofle, de lavande, de géranium, de cèdre, de coriandre, de genévrier, d'immortelle, de marjolaine ...;
- des produits aromatiques de synthèses tels que par exemple, les esters aromatiques comme les acétates de benzyle, de linalyle, de terpenyle, de vetyvéryle, d'amyle, de bornyle, de cédryle, de géranyle, de phényléthyle, de paracrésyle, de styrallyle, les butyrates d'amyle , l'eugénol, le géraniol, l'alcool anisique, l'alcool cinnamique, l'alcool styrallique, les aldéhydes tels que les aldéhydes octylique, nonylique, décylique, undécylénique, laurique, myristique, cétylique, stéarique, benzoique, anisique, le camphre synthétique, le limonène ;
- des parfums naturels ou de synthèse ;
- des adjuvants cosmétiques lipophiles ;
- des glycolipides comme par exemple les sophoroses lipides ;
- des agents conservateurs comme les méthyl, éthyl, propyl et butyl esters de l'acide p-hydroxybenzoïque, le benzoate de sodium, le GERMABEN® ou tout agent chimique évitant la prolifération bactérienne ou des moisissures et utilisés traditionnellement dans des compositions cosmétiques sont généralement introduits dans ces compositions à hauteur de 0,01 à 3% en poids ;
- des filtres solaires organiques actifs dans l'UV-A et/ou l'UV-B pour protéger la peau ou les cheveux des agressions du soleil et des rayons UV, comme les composés autorisés dans la directive Européenne N° 76/768/CEE, ses annexes et les modifications ultérieures de cette directive ;
- des antioxydants ;
- des agents répulsifs contre les insectes ;
- des vitamines ;
- des colorants ;
- des matières actives phytosanitaires comme les herbicides, les fongicides et les insecticides, tels que ceux décrits dans THE PESTICIDE MANUAL (9° édition, C.R. WORKLING et R.J. HANCE, éditeurs, publié par The British Crop Protection Council). Les adjuvants de formulation permettent de plus de faciliter la pénétration des pesticides dans la plante ce qui augmente l'efficacité des pesticides et permet ainsi de réduire leur dose d'utilisation ;
- de l'ester méthylique de colza ;
- des protéines ;
- des ingrédients pharmaceutiques lipophiles...

L'augmentation de la solubilité des molécules lipophiles dans l'eau est due à l'incorporation des produits lipophiles dans les micelles des tensioactifs ou leur incorporation au sein d'une organisation moléculaire moins ordonnée formant un milieu hydrotrope.

On préfère les compositions selon l'invention comprenant :
- 10% à 60% d'adjuvant suivant l'invention,
- 40% à 90% de tensioactifs non ioniques comme ceux cités précédemment.

On préfère tout particulièrement, en raison notamment de leur efficacité, de leur bonne biodégradabilité, de leur faible pouvoir irritant et de leur bonne écotoxicité les compositions suivant l'invention comprenant :
- 10% à 60% d'adjuvant suivant l'invention,
- 40% à 90% de polyglycosides d'alkyle de formule VII décrits dans le brevet EP 0 699 472 :

   R⁵-O(A₁)_{f}(A₂)_{g}(A₃)ₕ(A₄)ᵢ(A₅)ⱼ (VII)
dans lesquelles A₁, A₂, A₃, A₄, A₅ sont indépendamment les uns des autres, des restes d'un ose choisi parmi les hexoses et plus particulièrement le D-glucose ; les pentoses, ces derniers étant choisis préférentiellement parmi l'arabinose et le xylose ; f, g, h, i et j étant égaux à 0 ou à 1, la somme de f, g, h, i et j étant au moins égale à 1 ; R⁵ étant un radical linéaire ou ramifié alkyle de 6 à 22 atomes de carbone, hydrocarboné ayant de 1 à 4 insaturations éthyléniques et de 6 à 22 atomes de carbone ou l'un de ces radicaux substitués par 1 à 3 substituants sur des atomes de carbone différents choisis parmi hydroxy, halogène et trifluorométhyle. On préfère tout particulièrement les polyglycosides d'alkyle ayant de 8 à 16 atomes de carbone sur le radical alcoyle R⁵ ; et notamment, les mélanges de polyglycosides d'octyle et de décyle, les mélanges de polyglycosides de décyle et de dodécyle, les mélanges de polyglycosides de dodécyle et de tétradécyle, les mélanges de polyglycosides d'octyle, de décyle, de dodécyle et de tétradécyle et les mélanges de polyglycosides de dodécyle, de tétradécyle et d'hexadécyle.

La présente invention a également pour objet une composition limpide comprenant en poids :
- 0,5 à 5% d'adjuvant suivant l'invention,
- 2 à 7% de polyglycosides d'alkyle de formule VII comportant de 8 à 14 atomes de carbone sur la chaîne alkyle R⁵,
- 1 à 10 % d'alcanols linéaires ou ramifiés présentant de 2 à 5 atomes de carbone ou leurs mélanges,
- 0,1 à 3% de substances actives lipophile à solubiliser.

A titre d'exemple d'alcanols linéaires ou ramifiés présentant de 2 à 5 atomes de carbone, on peut citer l'éthanol, le 2-propanol, le n-butanol, le 2-méthyl propanol, le 2-méthyl butanol, le 3-méthyl butanol, le n-pentanol, les alcanols contenus dans les huiles de Fusel.

Les substances actives lipophiles à solubiliser peuvent être :
- des huiles essentielles telles que par exemple, les huiles de pin maritime ou sylvestre, les huiles d'agrumes tels que le citron, le pamplemousse, l'orange, la mandarine, les huiles de céréales telles que l'huile de gluten de blé, l'huile de germe de blé, les huiles d'anis, d'amande amère, de bouleau, de camomille, de bergamote, de cannelle, de citronnelle, de plantes aromatiques telles que le thym blanc, le thym rouge, le romarin, la menthe, l'eucalyptus, le basilic, l'estragon, le laurier, l'origan, la verveine, les huiles de genièvre, de girofle, de lavande, de géranium, de cèdre, de coriandre, de genévrier, d'immortelle, de marjolaine ;
- des produits aromatiques de synthèses tels que par exemple, les esters aromatiques comme les acétates de benzyle, de linalyle, de terpenyle, de vetyvéryle, d'amyle, de bornyle, de cédryle, de géranyle, de phényléthyle, de paracrésyle, de styrallyle, les butyrates d'amyle , l'eugénol, le géraniol, l'alcool anisique, l'alcool cinnamique, l'alcool styrallique, les aldéhydes tels que les aldéhydes octylique, nonylique, décylique, undécylénique, laurique, myristique, cétylique, stéarique, benzoique, anisique, le camphre synthétique, le limonène ;
- des parfums naturels ou de synthèse ;
- des adjuvants cosmétiques lipophiles ;
- des glycolipides comme par exemple les sophoroses lipides ;
- des agents conservateurs comme les méthyl, éthyl, propyl et butyl esters de l'acide p-hydroxybenzoïque, le benzoate de sodium, le GERMABEN® ou tout agent chimique évitant la prolifération bactérienne ou des moisissures et utilisés traditionnellement dans des compositions cosmétiques sont généralement introduits dans ces compositions à hauteur de 0,01 à 3% en poids ;
- des filtres solaires organiques actifs dans l'UV-A et/ou l'UV-B pour protéger la peau ou les cheveux des agressions du soleil et des rayons UV, comme les composés autorisés dans la directive Européenne N° 76/768/CEE, ses annexes et les modifications ultérieures de cette directive ;
- des agents répulsifs contre les insectes ;
- des vitamines ;
- des colorants ;
- des matières actives phytosanitaires comme les herbicides, les fongicides et les insecticides, tels que ceux décrits dans THE PESTICIDE MANUAL (9° édition, C.R. WORKLING et R.J. HANCE, éditeurs, publié par The British Crop Protection Council) ;
- de l'ester méthylique de colza ;
- des protéines ;
- des ingrédients pharmaceutiques lipophiles...

On préfère tout particulièrement les compositions selon l'invention comprenant en poids :
- 0,5 à 5% d'adjuvant selon l'invention,
- 1 à 10% de polyglycosides d'alkyle comportant de 8 à 14 atomes de carbone sur la chaîne alkyle,
- 1 à 10% d'alcanols linéaires ou ramifiés présentant de 2 à 5 atomes de carbone ou leurs mélanges,
- 0,1 à 2% d'huile essentielle,
- 0 à 0,5% d'agent conservateur comme les méthyl, éthyl, propyl et butyl esters de l'acide p-hydroxybenzoïque, le benzoate de sodium, le GERMABEN® ou tout agent chimique évitant la prolifération bactérienne ou des moisissures et utilisés traditionnellement dans des compositions cosmétiques et détergentes.

Ces compositions sont particulièrement efficaces pour le lavage des surfaces lisses et constituent des détergents multi-usages performants, biodégradables et respectueux de l'environnement.

D'une manière générale les adjuvants selon l'invention peuvent être présents dans les formulations comme les détergents en poudre ou liquide, les lotions moussantes ou non moussantes, les émulsions de consistance liquide ou semi-liquide telles que des laits obtenus par dispersion d'une phase grasse dans une phase aqueuse ou inversement, les suspensions ou les émulsions de consistance molle du type crèmes ou pommades, les gels ou encore les préparations solides telles que les sticks, les pains de nettoyage, les tampons imprégnés ou encore les masques hydratants.

Les adjuvants selon l'invention peuvent notamment être présents dans :
- les détergents ménagers et industriels, comme les nettoyants tout usage , les produits de lavage de la vaisselle, des sols, des vitres, des sanitaires, du textile;
- les formulations cosmétiques comme les lotions, les laits de toilettes, les compositions démaquillantes, les crèmes de soins ou les crèmes ou les lotions de protection contre le soleil et le rayonnement ultraviolet, les mousses, les gels coiffants ou toute formulation utilisée pour le coiffage ou pour faciliter le peignage des cheveux, les shampooings pour cheveux ou le corps, les gels de nettoyage du visage ou du corps, les savons liquides, les compositions moussantes pour le bain, les formulations utilisées pour le nettoyage des dents ou de la cavité buccale comme les bains de bouche ou les dentifrices, les savons ou les pains de toilette.
- Les formulations phytosanitaires.

Les exemples suivants ont pour but d'illustrer la présente invention.

### Exemple 1

### Synthèse d'adjuvant de solubilisation à partir de D-xylose et d'huiles de Fusel

1277 g d'huile de Fusel de composition suivante :

| **Constituant** | **%** |
|---|---|
| **Eau** | 10,0 |
| **Ethanol** | 6,0 |
| **2-propanol** | 0,2 |
| **1-propanol** | 2,1 |
| **2-méthyl-propanol** | 9,3 |
| **1-butanol** | 0,3 |
| **3-méthyl butanol** | 45,0 |
| **2-méthyl-butanol** | 20,3 |
| **Impuretés** | 6,8 |

sont portés à ébullition dans un bouilleur surmonté d'un colonne de rectification. On recueille dans un premier temps une fraction **A** présentant des points d'ébullition inférieurs à 100°C (204 g), puis dans un second temps une fraction **B** présentant des points d'ébullition compris entre 100 et 140°C (994 g) de composition suivante :

| **Constituant** | **%** |
|---|---|
| **Eau** | 6 |
| **Ethanol** | 3,2 |
| **2-propanol** | 0,2 |
| **1-propanol** | 2 |
| **2-méthyl-propanol** | 10,1 |
| **1-butanol** | 0,3 |
| **3-méthyl-butanol** | 55,3 |
| **2-méthyl-butanol** | 21 |
| **Impuretés** | 1,9 |

170g de D-xylose sont placés dans 630 g de la fraction **B** contenant 3,4 g d'acide sulfurique. Le milieu réactionnel est maintenu sous pression réduite pendant 3 heures à 100°C. L'eau est éliminée par distillation azéotropique au cours de la réaction. L'acidité du milieu est neutralisée par une solution aqueuse de soude à 30,5% jusqu'à pH 7 à 8. Les alcools de Fusel en excès sont évaporés sous pression réduite à 80°C. Le résidu (244 g) est solubilisé dans 100 g d'eau osmosée. Les xylosides d'alkyle obtenus sont décolorés en présence de 5 g d'eau oxygénée à 50% à pH neutre.

### Exemple 2

### Synthèse d'adjuvant à partir de L-arabinose et d'huiles de Fusel

1441 g d'huile de Fusel de composition suivante :

| **Constituant** | **%** |
|---|---|
| **Eau** | 16,1 |
| **Ethanol** | 22,6 |
| **2-propanol** | 0,2 |
| **1-propanol** | 2,6 |
| **2-méthyl-propanol** | 6,7 |
| **1-butanol** | 0,3 |
| **3-méthyl butanol** | 23,0 |
| **2-méthyl-butanol** | 10,5 |
| **Impuretés** | 18,0 |

sont portés à ébullition dans un bouilleur surmonté d'un colonne de rectification. On recueille dans un premier temps une fraction **A** présentant des points d'ébullition inférieurs à 100°C (594 g), puis dans un second temps une fraction **B** présentant des points d'ébullition compris entre 100 et 140°C qui décante. La phase supérieure **C** (582 g) présente la composition suivante :

| **Constituant** | **%** |
|---|---|
| **Eau** | 9 ,9 |
| **Ethanol** | 10,8 |
| **2-propanol** | 0 |
| **1-propanol** | 2,8 |
| **2-méthyl-propanol** | 9,2 |
| **1-butanol** | 0,6 |
| **3-méthyl-butanol** | 40,8 |
| **2-méthyl-butanol** | 18,4 |
| **Impuretés** | 7,5 |

200 g de L-arabinose sont placés dans 774 g de la fraction **C** contenant 4 g d'acide sulfurique. Le milieu réactionnel est maintenu sous pression réduite pendant 3 heures à 100°C. L'eau est éliminée par distillation azéotropique au cours de la réaction. L'acidité du milieu est neutralisée par une solution aqueuse de soude à 30,5% jusqu'à pH 7 à 8. Les alcools de Fusel en excès sont évaporés sous pression réduite à 80°C. Le résidu (282 g) est solubilisé dans 120 g d'eau osmosée. Les arabinosides d'alkyle obtenus sont décolorés en présence de 5 g d'eau oxygénée à 50% à pH neutre.

### Exemple 3

### Synthèse d'adjuvant à partir de D-glucose et d'huiles de Fusel

1277 g d'huile de Fusel de composition suivante :

| **Constituant** | **%** |
|---|---|
| **Eau** | 10,0 |
| **Ethanol** | 6,0 |
| **2-propanol** | 0,2 |
| **1-propanol** | 2,1 |
| **2-méthyl-propanol** | 9,3 |
| **1-butanol** | 0,3 |
| **3-méthyl butanol** | 45,0 |
| **2-méthyl-butanol** | 20,3 |
| **Impuretés** | 6,8 |

sont portés à ébullition dans un bouilleur surmonté d'un colonne de rectification. On recueille dans un premier temps une fraction **A** présentant des points d'ébullition inférieurs à 100°C (204 g), puis dans un second temps une fraction **B** présentant des points d'ébullition compris entre 100 et 140°C (994 g) de composition suivante :

| **Constituant** | **%** |
|---|---|
| **Eau** | 6 |
| **Ethanol** | 3,2 |
| **2-propanol** | 0,2 |
| **1-propanol** | 2 |
| **2-méthyl-propanol** | 10,1 |
| **1-butanol** | 0,3 |
| **3-méthyl-butanol** | 55,3 |
| **2-méthyl-butanol** | 21 |
| **Impuretés** | 1,9 |

200 g de D-glucose monohydraté sont placés dans 562 g de la fraction **B** contenant 3,6 g d'acide sulfurique. Le milieu réactionnel est maintenu sous pression réduite pendant 3 heures à 100°C. L'eau est éliminée par distillation azéotropique au cours de la réaction. Après filtration, l'acidité du milieu est neutralisée par une solution aqueuse de soude à 30,5% jusqu'à pH 7 à 8. Les alcools de Fusel en excès sont évaporés sous pression réduite à 80°C. Le résidu (244 g) est solubilisé dans 100 g d'eau osmosée. Les glucosides d'alkyle obtenus sont décolorés en présence de 5 g d'eau oxygénée à 50% à pH neutre.

### Exemple 4

### Synthèse d'adjuvant à partir de mélanges de D-xylose, L-arabinose et de D-glucose et d'huiles de Fusel

1277 g d'huile de Fusel de composition suivante :

| **Constituant** | **%** |
|---|---|
| **Eau** | 10,0 |
| **Ethanol** | 6,0 |
| **2-propanol** | 0,2 |
| **1-propanol** | 2,1 |
| **2-méthyl-propanol** | 9,3 |
| **1-butanol** | 0,3 |
| **3-méthyl butanol** | 45,0 |
| **2-méthyl-butanol** | 20,3 |
| **Impuretés** | 6,8 |

sont portés à ébullition dans un bouilleur surmonté d'un colonne de rectification. On recueille dans un premier temps une fraction **A** présentant des points d'ébullition inférieurs à 100°C (204g), puis dans un second temps une fraction **B** présentant des points d'ébullition compris entre 100 et 140°C (994 g) de composition suivante :

| **Constituant** | **%** |
|---|---|
| **Eau** | 6 |
| **Ethanol** | 3,2 |
| **2-propanol** | 0,2 |
| **1-propanol** | 2 |
| **2-méthyl-propanol** | 10,1 |
| **1-butanol** | 0,3 |
| **3-méthyl-butanol** | 55,3 |
| **2-méthyl-butanol** | 21 |
| **Impuretés** | 1,9 |

100 g de D-xylose, 100 g de L-arabinose et 100 g de D-glucose anhydre sont placés dans 1050 g de la fraction **B** contenant 6 g d'acide sulfurique. Le milieu réactionnel est maintenu sous pression réduite pendant 3 heures à 100°C. L'eau est éliminée par distillation azéotropique au cours de la réaction. L'acidité du milieu est neutralisée par une solution aqueuse de soude à 30,5% jusqu'à pH 7 à 8. Les alcools de Fusel en excès sont évaporés sous pression réduite à 80°C. Le résidu (420 g) est solubilisé dans 180 g d'eau osmosée. Les glycosides d'alkyle obtenus sont décolorés en présence de 12 g d'eau oxygénée à 50% à pH neutre.

### Exemple 5

### Synthèse d'adjuvant à partir de sirops de sucres issus de paille de blé et d'huiles de Fusel

1500 g d'huile de Fusel de composition suivante :

| **Constituant** | **%** |
|---|---|
| **Eau** | 10,0 |
| **Ethanol** | 6,0 |
| **2-propanol** | 0,2 |
| **1-propanol** | 2,1 |
| **2-méthyl-propanol** | 9,3 |
| **1-butanol** | 0,3 |
| **3-méthyl butanol** | 45,0 |
| **2-méthyl-butanol** | 20,3 |
| **Impuretés** | 6,8 |

sont portés à ébullition dans un bouilleur surmonté d'un colonne de rectification. On recueille dans un premier temps une fraction **A** présentant des points d'ébullition inférieurs à 100°C (242 g), puis dans un second temps une fraction **B** présentant des points d'ébullition compris entre 100 et 140°C (1160 g) de composition suivante :

| **Constituant** | **%** |
|---|---|
| **Eau** | 6 |
| **Ethanol** | 3,2 |
| **2-propanol** | 0,2 |
| **1-propanol** | 2 |
| **2-méthyl-propanol** | 10,1 |
| **1-butanol** | 0,3 |
| **3-méthyl-butanol** | 55,3 |
| **2-méthyl-butanol** | 21 |
| **Impuretés** | 1,9 |

150 g de sirop de sucres issus de paille de blé contenant 37,5 g d'eau, 79 g de D-xylose, 13,6 g de L-arabinose, 8 g de D-glucose et 4,5g de D-galactose et de D-mannose sont ajoutés goutte à goutte en 1 heure 30 dans 400 g de la fraction **B** contenant 2,25 g d'acide sulfurique à une température de l'ordre de 100 à 107°C. L'eau est éliminée au cours de la réaction par distillation azéotropique. Après l'addition du sirop de sucres, la pression du milieu réactionnel est abaissée progressivement entre 300 et 900 mb à la même température afin d'éliminer les traces d'eau. L'acidité du milieu est ensuite neutralisée par une solution aqueuse de soude à 30,5% jusqu'à pH 7 à 8. Les alcools de Fusel en excès sont évaporés sous pression réduite à 80°C. Le résidu (155 g) est solubilisé dans 65 g d'eau osmosée. Les glycosides d'alkyle obtenus sont décolorés en présence de 5 g d'eau oxygénée à 50% à pH neutre.

### Exemple 6

### Synthèse d'adjuvant à partir de sirops de sucres extraits de son désamylacé de blé et d'huiles de Fusel

1277 g d'huile de Fusel de composition suivante :

| **Constituant** | **%** |
|---|---|
| **Eau** | 10,0 |
| **Ethanol** | 6,0 |
| **2-propanol** | 0,2 |
| **1-propanol** | 2,1 |
| **2-méthyl-propanol** | 9,3 |
| **1-butanol** | 0,3 |
| **3-méthyl butanol** | 45,0 |
| **2-méthyl-butanol** | 20,3 |
| **Impuretés** | 6,8 |

sont portés à ébullition dans un bouilleur surmonté d'un colonne de rectification. On recueille dans un premier temps une fraction **A** présentant des points d'ébullition inférieurs à 100°C (204 g), puis dans un second temps une fraction **B** présentant des points d'ébullition compris entre 100 et 140°C (994 g) de composition suivante :

| **Constituant** | **%** |
|---|---|
| **Eau** | 6 |
| **Ethanol** | 3,2 |
| **2-propanol** | 0,2 |
| **1-propanol** | 2 |
| **2-méthyl-propanol** | 10,1 |
| **1-butanol** | 0,3 |
| **3-méthyl-butanol** | 55,3 |
| **2-méthyl-butanol** | 21 |
| **Impuretés** | 1,9 |

200 g de sirop de sucres issus de son de blé désamylacé contenant 50 g d'eau, 72 g de D-xylose, 42 g de L-arabinose, 10,4 g de D-glucose et 3,7 g de D-galactose et D-mannose sont ajoutés goutte à goutte en 1 heure 30 dans 525 g de la fraction **B** contenant 3 g d'acide sulfurique à une température de l'ordre de 100 à 105°C. L'eau est éliminée au cours de la réaction par distillation azéotropique. Après l'addition du sirop de sucres, la pression du milieu réactionnel est abaissée progressivement entre 300 et 900 mb à la même température afin d'éliminer les traces d'eau. L'acidité du milieu est ensuite neutralisée par une solution aqueuse de soude à 30,5% jusqu'à pH 7 à 8. Les alcools de Fusel en excès sont évaporés sous pression réduite à 80°C. Le résidu (207 g) est solubilisé dans 90 g d'eau osmosée. Les glycosides d'alkyle obtenus sont décolorés en présence de 6 g d'eau oxygénée à 50% à pH neutre.

### Exemple 7

### Synthèse d'adjuvant à partir de sirops de sucres extraits de son brut de blé et d'huiles de Fusel

1277 g d'huile de Fusel de composition suivante :

| **Constituant** | **%** |
|---|---|
| **Eau** | 10,0 |
| **Ethanol** | 6,0 |
| **2-propanol** | 0,2 |
| **1-propanol** | 2,1 |
| **2-méthyl-propanol** | 9,3 |
| **1-butanol** | 0,3 |
| **3-méthyl butanol** | 45,0 |
| **2-méthyl-butanol** | 20,3 |
| **Impuretés** | 6,8 |

sont portés à ébullition dans un bouilleur surmonté d'un colonne de rectification. On recueille dans un premier temps une fraction **A** présentant des points d'ébullition inférieurs à 100°C (204 g), puis dans un second temps une fraction **B** présentant des points d'ébullition compris entre 100 et 140°C (994 g)de composition suivante :

| **Constituant** | **%** |
|---|---|
| **Eau** | 6 |
| **Ethanol** | 3,2 |
| **2-propanol** | 0,2 |
| **1-propanol** | 2 |
| **2-méthyl-propanol** | 10,1 |
| **1-butanol** | 0,3 |
| **3-méthyl-butanol** | 55,3 |
| **2-méthyl-butanol** | 21 |
| **Impuretés** | 1,9 |

200 g de sirop de sucres issus de son brut de blé contenant 50 g d'eau, 34,5 g de D-xylose, 20 g de L-arabinose, 71 g de D-glucose et 2 g de D-galactose et de D-mannose sont ajoutés goutte à goutte en 1 heure 30 dans 470 g de la fraction **B** contenant 3 g d'acide sulfurique à une température de l'ordre de 100 à 105°C. L'eau est éliminée au cours de la réaction par distillation azéotropique. Après l'addition du sirop de sucres, la pression du milieu réactionnel est abaissée progressivement entre 300 et 900 mb à la même température afin d'éliminer les traces d'eau. L'acidité du milieu est ensuite neutralisée par une solution aqueuse de soude à 30,5% jusqu'à pH 7 à 8. Les alcools de Fusel en excès sont évaporés sous pression réduite à 80°C. Le résidu (202 g) est solubilisé dans 90 g d'eau osmosée. Les glycosides d'alkyle obtenus sont décolorés en présence de 8 g d'eau oxygénée à 50% à pH neutre.

### Exemple 8

### Synthèse d'adjuvant à partir de D-xylose et d'huiles de Fusel

150 g de D-xylose sont placés dans 740 g d'huile de Fusel de composition suivante :

| **Constituant** | **%** |
|---|---|
| **Eau** | 10,0 |
| **Ethanol** | 6,0 |
| **2-propanol** | 0,2 |
| **1-propanol** | 2,1 |
| **2-méthyl-propanol** | 9,3 |
| **1-butanol** | 0,3 |
| **3-méthyl butanol** | 45,0 |
| **2-méthyl-butanol** | 20,3 |
| **Impuretés** | 6,8 |

et contenant 7,5 g d'acide sulfurique. Le milieu réactionnel est maintenu sous pression réduite pendant 1 heure à 110°C. L'eau est éliminée par distillation azéotropique au cours de la réaction. L'acidité du milieu est neutralisée par une solution aqueuse de soude à 30,5% jusqu'à pH 7 à 8. Les alcools de Fusel en excès sont évaporés sous pression réduite à 80°C. Le résidu (200 g) est solubilisé dans 100 g d'eau osmosée. Les xylosides d'alkyle obtenus sont décolorés en présence de 10 g d'eau oxygénée à 50% à pH neutre.

### Exemple 9

### Adjuvant de solubilisation

50 g d'adjuvant de solubilisation de l'exemple 1 sont mélangés sous agitation à 25 g d'adjuvant de solubilisation de l'exemple 2 et 25 g d'adjuvant de solubilisation de l'exemple 3 afin d'obtenir un nouvel adjuvant de solubilisation.

### Exemple 10

### Adjuvant de solubilisation

85 g d'adjuvant de solubilisation de l'exemple 1 sont mélangés sous agitation à 15 g d'adjuvant de solubilisation de l'exemple 3 afin d'obtenir un nouvel adjuvant de solubilisation.

### Exemple 11

### Adjuvant de solubilisation

31 g d'adjuvant de solubilisation de l'exemple 1 sont mélangés sous agitation à 69 g d'adjuvant de solubilisation de l'exemple 3 afin d'obtenir un nouvel adjuvant de solubilisation.

### Exemple 12

### Composition solubilisante

30 g d'adjuvant de solubilisation de l'exemple 10 sont mélangés sous agitation à 70 g de polyglycosides d'alkyle tels que préparés dans l'exemple 9 du brevet EP 0 699 472 afin d'obtenir une composition solubilisante.

### Exemple 13

### Composition solubilisante

30 g d'adjuvant de solubilisation de l'exemple 11 sont mélangés sous agitation à 70 g de polyglycosides d'alkyle tels que préparés dans l'exemple 9 du brevet EP 0 699 472 afin d'obtenir une composition solubilisante.

### Exemple 14

### Composition solubilisante

40 g d'adjuvant de solubilisation de l'exemple 5 sont mélangés sous agitation à 60 g de polyglycosides d'alkyle tels que préparés dans l'exemple 6 du brevet EP 0 699 472 afin d'obtenir une composition solubilisante.

### Exemple 15

### Composition solubilisante

25 g d'adjuvant de solubilisation de l'exemple 6 sont mélangés sous agitation à 75 g de polyglycosides d'alkyle tels que préparés dans l'exemple 9 du brevet EP 0 699 472 afin d'obtenir une composition solubilisante.

### Exemple 16

### Composition solubilisante

30 g d'adjuvant de solubilisation de l'exemple 1 sont mélangés sous agitation à 60 g de polyglycosides d'alkyle tels que préparés dans l'exemple 9 du brevet EP 0 699 472 afin d'obtenir une composition solubilisante.

### Exemple 17

### Solubilisation d'un parfum citron de synthèse

100 µl de parfum de synthèse citron 19-11454 de Huber the Nose insoluble dans l'eau sont mis en suspension dans 5 ml d'eau osmosée contenant la composition solubilisante de l'exemple 12. 8,4 g de composition solubilisante de l'exemple 12 permettent la solubilisation de 1 g de parfum. On obtient alors une solution parfaitement limpide après 1 minute d'agitation.

### Exemple 18

### Solubilisation d'un parfum marine de synthèse

100 µl de parfum marine 813417F insoluble dans l'eau sont mis en suspension dans 5 ml d'eau osmosée contenant la composition solubilisante de l'exemple 12. 7,4 g de composition solubilisante de l'exemple 12 permettent la solubilisation de 1 g de parfum. On obtient alors une solution parfaitement limpide après 1 minute d'agitation.

### Exemple 19

### Solubilisation d'ester méthylique de colza

100 µl d'ester méthylique de colza (TOTAL RADIA 7961) insoluble dans l'eau sont mis en suspension dans 5 ml d'eau osmosée contenant la composition solubilisante de l'exemple 13. 5,4 g de composition solubilisante de l'exemple 13 permettent la solubilisation de 1 g d'ester méthylique de colza. On obtient alors une solution parfaitement limpide après 1 minute d'agitation.

### Exemple 20

### Solubilisation de 1-décanol

100 µl de décanol insoluble dans l'eau sont mis en suspension dans 5 ml d'eau osmosée contenant la composition solubilisante de l'exemple 13. 6,5 g de composition solubilisante de l'exemple 13 permettent la solubilisation de 1 g de décanol. On obtient alors une solution parfaitement limpide après 1 minute d'agitation.

### Exemple 21

### Solubilisation de myristate d'isopropyle

100 µl de myristate d'isopropyle insoluble dans l'eau sont mis en suspension dans 5 ml d'eau osmosée contenant la composition solubilisante de l'exemple 12. 10 g de composition solubilisante de l'exemple 12 permettent la solubilisation de 1 g de myristate d'isopropyle. On obtient alors une solution parfaitement limpide après 1 minute d'agitation.

### Exemple 22

### Solubilisation d'huile essentielle de romarin

100 µl d'huile essentielle de romarin insoluble dans l'eau sont mis en suspension dans 5 ml d'eau osmosée contenant la composition solubilisante de l'exemple 12. 5,9 g de composition solubilisante de l'exemple 12 permettent la solubilisation de 1 g d'huile essentielle de romarin. On obtient alors une solution parfaitement limpide après 1 minute d'agitation.

### Exemple 23

### Composition limpide

Une composition limpide est réalisée par mélange de :
- 7,1 g de composition solubilisante de l'exemple 12
- 3 g d'éthanol
- 0,3 g de parfum citron citron 19-11454 de Huber the Nose
- 0,4 g de conservateur (phénonip®)
- Eau osmosée qsp 100 g

### Exemple 24

### Composition limpide détergente

Une composition limpide est réalisée par mélange de :
- 8,6 g de composition solubilisante de l'exemple 15
- 4 g d'éthanol
- 0,5 g d'huile essentielle de pin 34230 (OSF)
- 0,4 g de conservateur (phénonip®)
- Eau osmosée qsp 100 g.

Cette composition présente d'excellentes propriétés détergentes multi-usages notamment pour le lavage des surfaces lisses.

### Exemple 25

### Composition limpide détergente

Une composition limpide est réalisée par mélange de :
- 8,1 g de composition solubilisante de l'exemple 15
- 0,3 g de dodécyl-sulfate de sodium
- 3 g d'éthanol
- 0,5 g d'huile essentielle de pin 34230 (OSF)
- 0,4 g de conservateur (phénonip®)
- Eau osmosée qsp 100 g.

Cette composition présente d'excellentes propriétés détergentes multi-usages notamment pour le lavage des surfaces lisses.

### Exemple 26

### Composition limpide détergente

Une composition limpide est réalisée par mélange de :
- 8,6 g de composition solubilisante de l'exemple 15
- 3 g d'éthanol
- 0,5 g d'huile essentielle de citron 9286 (OSF)
- 0,4 g de conservateur (phénonip®)
- Eau osmosée qsp 100 g.

Cette composition présente d'excellentes propriétés détergentes multi-usages notamment pour le lavage des surfaces lisses.

### Exemple 27

### Composition limpide détergente

Une composition limpide est réalisée par mélange de :
- 8,1 g de composition solubilisante de l'exemple 15
- 0,3 g de dodécyl-sulfate de sodium
- 3 g d'éthanol
- 0,5 g d'huile essentielle de citron 9286 (OSF)
- 0,4 g de conservateur (phénonip®)
- Eau osmosée qsp 100 g.

Cette composition présente d'excellentes propriétés détergentes multi-usages notamment pour le lavage des surfaces lisses.

## Revendications

1. Procédé de préparation d'adjuvant de solubilisation **caractérisé en ce qu'**il consiste à mettre en contact des huiles de Fusel avec un ou plusieurs sucres réducteurs en présence d'un catalyseur acide, à une température comprise entre 50 et 130°C et en éliminant l'eau du milieu réactionnel jusqu'à obtention d'une solution de glycosides d'alkyle et à séparer les glycosides de cette solution.

2. Procédé suivant la revendication 1, **caractérisé en ce qu'**il consiste, avant la mise en contact avec un ou plusieurs sucres réducteurs, à éliminer les fractions lourdes des huiles de Fusel qui présentent des points d'ébullition supérieurs à 140°C.

3. Procédé suivant l'une des revendication 1 à 2, **caractérisé en ce qu'**il consiste, avant la mise en contact avec un ou plusieurs sucres réducteurs, à éliminer les fractions lourdes des huiles de Fusel qui présentent des points d'ébullition supérieurs à 140°C par distillation.

4. Procédé suivant l'une des revendication 1 à 3, **caractérisé en ce qu'**il consiste, avant la mise en contact avec un ou plusieurs sucres réducteurs, à éliminer les fractions légères des huiles de Fusel qui présentent des points d'ébullition inférieurs à 100°C.

5. Procédé suivant l'une des revendication 1 à 4, **caractérisé en ce qu'**il consiste, avant la mise en contact avec un ou plusieurs sucres réducteurs, à éliminer les fractions légères des huiles de Fusel qui présentent des points d'ébullition inférieurs à 100°C par distillation.

6. Procédé suivant l'une des revendication 1 à 5, **caractérisé en ce qu'**il consiste, à utiliser comme sucres réducteurs des pentoses choisis parmi le L-arabinose ou le D-xylose.

7. Procédé suivant l'une des revendication 1 à 5, **caractérisé en ce qu'**il consiste, à utiliser comme sucres réducteurs du glucose.

8. Procédé suivant l'une des revendication 1 à 5, **caractérisé en ce qu'**il consiste, à utiliser comme sucres réducteurs des mélanges de sucres comprenant en poids de 25% à 98% de pentoses, de préférence 60 à 100 % et plus particulièrement 90 à 100% choisis parmi le L-arabinose ou le D-xylose et 2% à 75%, de préférence 0 à 40% et plus particulièrement 0 à 10% d'hexoses choisis parmi le D-glucose, le D-galactose et le D-mannose.

9. Adjuvant **caractérisé en ce qu'**il comprend en poids à l'exception des impuretés :
- de 0% à 20 % d'un mélange de polyglycosides de formule (I):
H₃C-CH₂-O(G₁)ₐ(G₂)_{b}(G₃)_{b}(G₄)_{d}(G₅)ₑ (I)
- de 0% à 5% d'un mélange de polyglycosides de formule (II) :
H₃C-CH₂-CH₂-O(G₁)ₐ(G₂)_{b}(G₃)_{c}(G₄)_{d}(G₅)ₑ (II)
- de 0% à 15% d'un mélange de polyglycosides de formule (III) : 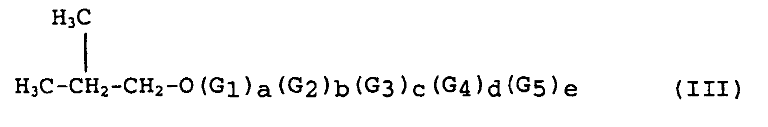
- de 20% à 80% d'un mélange de polyglycosides de formule (IV): 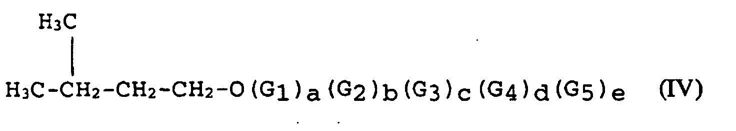
- de 10% à 40% d'un mélange de polyglycosides de formule (V) : 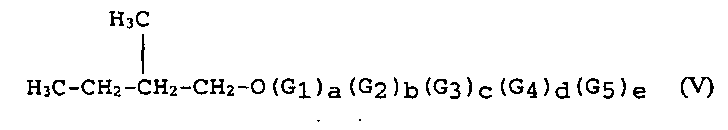
dans lesquelles G₁, G₂, G₃, G₄, G₅ sont indépendamment les uns des autres, des restes d'un ose choisi préférentiellement parmi les hexoses et les pentoses, ces derniers étant choisis préférentiellement parmi l'arabinose et le xylose ; a, b, c, d et e étant égaux à 0 ou à 1, la somme de a, b, c, d et e étant au moins égale à 1. L'ensemble des composés I, II, III, IV et V, hormis les impuretés et d'éventuels glycosides d'alkyle autres que les composés I, II, III, IV et V, représentent 100%.

10. Adjuvant suivant la revendication 9, **caractérisé en ce qu'**il comprend au moins en poids :
- de 0% à 20% d'un mélange de polyglycosides de formule (III),
- de 45% à 80% d'un mélange de polyglycosides de formule (IV),
- de 10% à 40% d'un mélange de polyglycosides de formule (V).

11. Adjuvant suivant la revendication 9, **caractérisé en ce qu'**il comprend au moins en poids :
- de 60% à 75% d'un mélange de polyglycosides de formule (IV),
- de 25% à 40% d'un mélange de polyglycosides de formule (V).

12. Adjuvant suivant l'une des revendications 9 à 11, **caractérisé en ce qu'**il soit dérivé de pentoses choisis parmi le L-arabinose ou le D-xylose.

13. Adjuvant suivant l'une des revendications 9 à 11, **caractérisé en ce qu'**il soit dérivé de D-glucose.

14. Adjuvant suivant l'une des revendications 9 à 11, **caractérisé en ce qu'**il soit dérivé de mélanges de sucres comprenant en poids de 25% à 98% de pentoses, de préférence 60 à 100 % et plus particulièrement 90 à 100% choisis parmi le L-arabinose ou le D-xylose et 2% à 75%, de préférence 0 à 40% et plus particulièrement 0 à 10% d'hexoses choisis parmi le D-glucose, le D-galactose et le D-mannose.

15. Composition **caractérisée en ce qu'**elle comprend au moins en poids :
- 10% à 60% d'adjuvant suivant l'une des revendications 9 à 14
- 40% à 90% de tensioactifs non ioniques, anioniques, amphotères, cationiques ou leurs mélanges.

16. Composition suivant la revendication 15 **caractérisée en ce qu'**elle comprend en poids :
- 10% à 60% d'adjuvant suivant l'une des revendications 9 à 14
- 40% à 90% de tensioactifs non ioniques.

17. Composition suivant l'une des revendications 15 à 16 **caractérisée en ce qu'**elle comprend en poids :
- 10% à 60% d'adjuvant suivant l'une des revendications 9 à 14
- 40% à 90% de polyglycosides d'alkyle comportant de 8 à 22 atomes de carbone sur la chaîne alkyle.

18. Composition **caractérisée en ce qu'**elle comprend en poids :
- 0,5 à 5% d'adjuvant suivant l'une des revendications 9 à 14,
- 2 à 7% de polyglycosides d'alkyle comportant de 8 à 14 atomes de carbone sur la chaîne alkyle,
- 1 à 10 % d'alcanols linéaires ou ramifiés présentant de 2 à 5 atomes de carbone ou leurs mélanges,
- 0,1 à 3% de substances actives lipophiles à solubiliser.

19. Composition suivant la revendication 18 **caractérisée en ce qu'**elle comprend en poids :
- 0,5 à 5% d'adjuvant suivant l'une des revendications 7 à 9
- 1 à 10% de polyglycosides d'alkyle comportant de 8 à 14 atomes de carbone sur la chaîne alkyle,
- 1 à 10% d'alcanols linéaires ou ramifiés présentant de 2 à 5 atomes de carbone ou leurs mélanges,
- 0,1 à 2% d'huile essentielle,
- 0 à 0,5% d'agent conservateur.

20. Composition suivant la revendication 19 **caractérisée en ce que** l'huile essentielle est choisie parmi les huiles de pin, de citron, d'orange, de mandarine, de pamplemousse, de lavande, de menthe, de thym, de romarin, d'eucalyptus.

21. Utilisation des adjuvants selon les revendications 9 à 14 en cosmétique, dermo-cosmétique, pharmacie, dans les produits phytosanitaires.

## Claims

1. Process for preparing a solubilization adjuvant, which comprises placing fusel oils in contact with one or more reducing sugars in the presence of an acid catalyst, at a temperature of between 50°C and 130°C and while removing the water from the reaction medium until a solution of alkyl glycosides is obtained, and separating the glycosides from this solution.

2. Process according to Claim 1, which comprises, before the placing in contact with one or more reducing sugars, removing the heavy fractions from the fusel oils which have boiling points of greater than 140°C.

3. Process according to either of Claims 1 and 2, which comprises, before the placing in contact with one or more reducing sugars, removing the heavy fractions from the fusel oils which have boiling points of greater than 140°C, by distillation.

4. Process according to one of Claims 1 to 3, which comprises, before the placing in contact with one or more reducing sugars, removing the light fractions from the fusel oils which have boiling points of less than 100°C.

5. Process according to one of Claims 1 to 4, which comprises, before the placing in contact with one or more reducing sugars, removing the light fractions from the fusel oils which have boiling points of less than 100°C, by distillation.

6. Process according to one of Claims 1 to 5, which comprises using, as reducing sugars, pentoses chosen from L-arabinose and D-xylose.

7. Process according to one of Claims 1 to 5, which comprises using glucose as reducing sugar.

8. Process according to one of Claims 1 to 5, which comprises using, as reducing sugars, sugar mixtures comprising, on a weight basis, from 25% to 98%, preferably 60% to 100% and more particularly 90% to 100%, of pentoses chosen from L-arabinose and D-xylose, and 2% to 75%, preferably 0% to 40% and more particularly 0% to 10%, of hexoses chosen from D-glucose, D-galactose and D-mannose.

9. Adjuvant, which comprises, on a weight basis, with the exception of the impurities:
- from 0% to 20% of a mixture of polyglycosides of formula (I) :
H₃C-CH₂-O(G₁)ₐ(G₂)_{b}(G₃)_{c}(G₄)_{d}(G₅)ₑ (I)
- from 0% to 5% of a mixture of polyglycosides of formula (II):
H₃C-CH₂-CH₂-O(G₁)ₐ(G₂)_{b}(G₃)_{c}(G₄)_{d}(G₅)ₑ (II)
- from 0% to 15% of a mixture of polyglycosides of formula (III) : 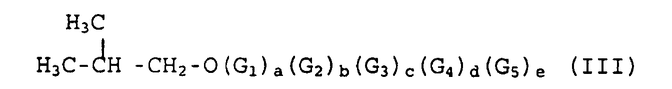
- from 20% to 80% of a mixture of polyglycosides of formula (IV): 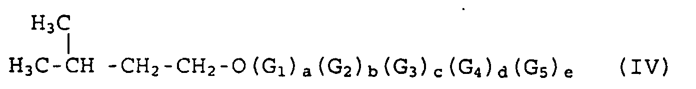
- from 10% to 40% of a mixture of polyglycosides of formula (V): 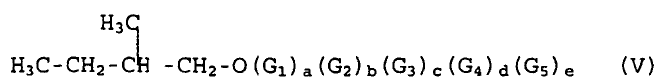 in which G₁, G₂, G₃, G₄ and G₅ are, independently of each other, residues of a saccharide preferentially chosen from hexoses and pentoses, these saccharides preferentially being chosen from arabinose and xylose; a, b, c, d and e being equal to 0 or 1, the sum of a, b, c, d and e being at least equal to 1. The combination of compounds I, II, III, IV and V, excluding the impurities and any alkyl glycosides other than the compounds I, II, III, IV and V, represents 100%.

10. Adjuvant according to Claim 9, which comprises at least, on a weight basis:
- from 0% to 20% of a mixture of polyglycosides of formula (III),
- from 45% to 80% of a mixture of polyglycosides of formula (IV),
- from 10% to 40% of a mixture of polyglycosides of formula (V).

11. Adjuvant according to Claim 9, which comprises at least, on a weight basis:
- from 60% to 75% of a mixture of polyglycosides of formula (IV),
- from 25% to 40% of a mixture of polyglycosides of formula (V).

12. Adjuvant according to one of Claims 9 to 11, which comprises it being derived from pentoses chosen from L-arabinose and D-xylose.

13. Adjuvant according to one of Claims 9 to 11, which comprises it being derived from D-glucose.

14. Adjuvant according to one of Claims 9 to 11, which comprises it being derived from sugar mixtures comprising, on a weight basis, from 25% to 98%, preferably 60% to 100% and more particularly 90% to 100%, of pentoses chosen from L-arabinose and D-xylose, and 2% to 75%, preferably 0% to 40% and more particularly 0% to 10%, of hexoses chosen from D-glucose, D-galactose and D-mannose.

15. Composition, which comprises at least, on a weight basis:
- 10% to 60% of adjuvant according to one of Claims 9 to 14
- 40% to 90% of nonionic, anionic, amphoteric or cationic surfactants, or mixtures thereof.

16. Composition according to Claim 15, which comprises, on a weight basis:
- 10% to 60% of adjuvant according to one of Claims 9 to 14
- 40% to 90% of nonionic surfactants.

17. Composition according to either of Claims 15 and 16, which comprises, on a weight basis:
- 10% to 60% of adjuvant according to one of Claims 9 to 14
- 40% to 90% of alkyl polyglycosides containing from 8 to 22 carbon atoms on the alkyl chain.

18. Composition, which comprises, on a weight basis:
- 0.5% to 5% of adjuvant according to one of Claims 9 to 14,
- 2% to 7% of alkyl polyglycosides containing from 8 to 14 carbon atoms on the alkyl chain,
- 1% to 10% of linear or branched alkanols containing from 2 to 5 carbon atoms, or mixtures thereof,
- 0.1% to 3% of lipophilic active substances to be dissolved.

19. Composition according to Claim 18, which comprises, on a weight basis:
- 0.5% to 5% of adjuvant according to one of Claims 7 to 9,
- 1% to 10% of alkyl polyglycosides containing from 8 to 14 carbon atoms on the alkyl chain,
- 1% to 10% of linear or branched alkanols containing from 2 to 5 carbon atoms, or mixtures thereof,
- 0.1% to 2% of essential oil,
- 0% to 0.5% of preserving agent.

20. Composition according to Claim 19, which comprises the essential oil being chosen from pine oil, lemon oil, orange oil, mandarin oil, grapefruit oil, lavender oil, mint oil, thyme oil, rosemary oil and eucalyptus oil.

21. Use of the adjuvants according to Claims 9 to 14 in cosmetics, dermocosmetics and pharmaceuticals, and in plant-protection products.

## Patentansprüche

1. Verfahren zur Herstellung eines Solubilisierungszusatzes, **dadurch gekennzeichnet, dass** es aufweist:
Fuselöle mit einem oder mehreren reduzierenden Zuckern in Gegenwart eines sauren Katalysators, bei einer Temperatur zwischen 50 und 130°C zu kontaktieren, das Wasser des Reaktionsmilieus bis zum Erhalt einer Alkylglycosidlösung zu entfernen und aus dieser Lösung die Glycoside abzutrennen.

2. Verfahren nach Anspruch 1, **dadurch gekennzeichnet, dass** es aufweist: vor Kontaktierung mit einem oder mehreren reduzierenden Zuckern die schweren Fraktionen der Fuselöle mit Siedepunkten über 140°C zu eliminieren.

3. Verfahren nach einem der Ansprüche 1 - 2, **dadurch gekennzeichnet, dass** es aufweist: vor Kontaktierung mit einem oder mehreren reduzierenden Zuckern die schweren Fraktionen der Fuselöle mit Siedepunkten über 140°C durch Destillation zu entfernen.

4. Verfahren nach einem der Ansprüche 1 - 3, **dadurch gekennzeichnet, dass** es aufweist: vor Kontaktierung mit einem oder mehreren reduzierenden Zuckern die leichten Fraktionen der Fuselöle mit Siedepunkten unter100°C zu entfernen.

5. Verfahren nach einem der Ansprüche 1 - 4, **dadurch gekennzeichnet, dass** es aufweist: vor Kontaktierung mit einem oder mehreren reduzierenden Zuckern die leichten Fraktionen der Fuselöle mit Siedepunkten unter 100°C durch Destillation zu entfernen.

6. Verfahren nach einem der Ansprüche 1 - 5, **dadurch gekennzeichnet, dass** es aufweist: als reduzierende Zucker Pentosen ausgewählt aus L-Arabinose oder D-Xylose, zu verwenden.

7. Verfahren nach einem der Ansprüche 1 - 5, **dadurch gekennzeichnet, dass** aufweist: als reduzierende Zucker Glucose zu verwenden.

8. Verfahren nach einem der Ansprüche 1 - 5, **dadurch gekennzeichnet, dass** es aufweist: als reduzierende Zucker Mischungen von Zuckern zu verwenden, die in Gew.% aufweisen: 25% - 98% Pentosen, bevorzugt 60 - 100 % und besonders bevorzugt 90 - 100% ausgewählt aus L-Arabinose oder D-Xylose und 2% - 75%, bevorzugt 0 - 40% und besonders bevorzugt 0 - 10% Hexosen ausgewählt aus D-Glucose, D-Galactose und D-Mannose.

9. Zusatz, **dadurch gekennzeichnet, dass** es mit Ausnahme von Verunreinigungen in Gew.% aufweist:
H₃C - CH₂ -O(G₁)ₐ(G₂)_{b}(G₃)_{c}(G₄)_{d}(G₅)ₑ (I)
0% - 20 % einer Mischung von Polyglycosiden der Formel (I)
0% - 5% einer Mischung von Polyglycosiden der Formel (II)
H₃C - CH₂- CH₂-0 (G₁)ₐ(G₂)_{b}(G₃)_{c}(G₄)_{d}(G₅)ₑ (II)
- 0% - 15% einer Mischung von Polyglycosiden der Formel (III): 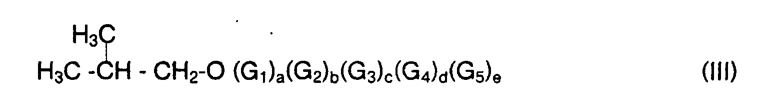
- 20% - 80% einer Mischung von Polyglycosiden der Formel (IV) 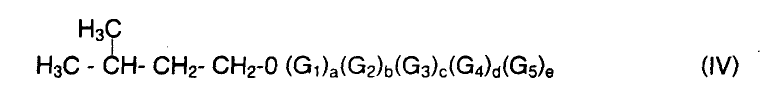 10% - 40% einer Mischung von Polyglycosiden der Formel (V) 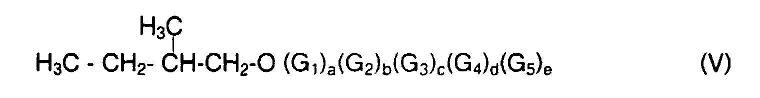 worin(G₁ G₂ G₃ G₄ G₅ unabhängig voneinander Reste von - osen, ausgewählt bevorzugt aus Hexosen und Pentosen, letztere bevorzugt ausgewählt aus Arabinose und Xylose, wobei a, b, c, d und e gleich 0 oder 1 sind, wobei die Summe von a, b, c, d und e mindestens gleich ist und die Gesamtheit der Zusammensetzungen I, II, III, IV et V, hormis zufälliger Verunreinigungen Alkylglycosiden, die nicht die Zusammensetzungen I, II, III, IV et V sind, 100% repräsentieren.

10. Zusatz nach Anspruch 9, **dadurch gekennzeichnet, dass** er mindestens in Gewichtsprozentsätzen:
- 0% - 20% einer Mischung von Polyglycosiden der Formel (III),
- 45% - 80% einer Mischung von Polyglycosiden der Formel (IV),
- 10% - 40% einer Mischung von Polyglycosiden der Formel (V).

11. Zusatz nach Anspruch 9, **dadurch gekennzeichnet, dass** er mindestens in Gew.% aufweist:
60% - 75% einer Mischung von Polyglycosiden der Formel (IV),
25% - 40% einer Mischung von Polyglycosiden der Formel (V).

12. Zusatz nach einem der Ansprüche 9 - 11, **dadurch gekennzeichnet, dass** er abgeleitet ist von Pentosen, ausgewählt aus L-Arabinose oder D-Xylose.

13. Zusatz nach einem der Ansprüche 9 - 11, **dadurch gekennzeichnet, dass** er abgeleitet ist aus D-Glucose.

14. Zusatz nach einem der Ansprüche 9 - 11, **dadurch gekennzeichnet, dass** er aus Mischungen von Zuckern, die aufweisen:
25 Gew.% - 98 Gew.% Pentosen, wovon bevorzugt 60 - 100 % und besonders bevorzugt 90 - 100% ausgewählt aus L-Arabinose oder D-Xylose und 2% - 75%, sind; und bevorzugt 0 - 40% und besonders bevorzugt 0 - 10 Gew.% Hexosen, ausgewählt aus D-Glucose, D-Galactose und D-Mannose, abgeleitet ist.

15. Zusammensetzung **dadurch gekennzeichnet, dass** sie mindestens - in Gew.%:
- 10% - 60% eines Zusatzes nach einem der Ansprüche 9 - 14
- 40% - 90% nicht ionischen, anionischen, amphoteren, kationischen Tensiden oder ihren Mischungen, aufweist.

16. Zusammensetzung nach Anspruch 15, **dadurch gekennzeichnet, dass** in Gew.% aufweist:
- 10% - 60% eines Zusatzes nach einem der Ansprüche 9 - 14
- 40% - 90% nicht ionische Tenside.

17. Zusammensetzung nach einem der Ansprüche 15 - 16, **dadurch gekennzeichnet, dass** sie in Gew.% aufweist:
- 10% - 60% eines Zusatzes nach einem der Ansprüche 9 - 14
- 40% - 90% Alkylpolyglycoside mit 8 - 22 Kohlenstoffatomen in der Alkylkette.

18. Zusammensetzung, **dadurch gekennzeichnet, dass** sie in Gew.% aufweist:
- 0,5 - 5% eines Zusatzes nach einem der Ansprüche 9 - 14,
- 2 - 7% Alkylpolyglycoside mit 8 - 14 Kohlenstoffatomen in der Alkylkette,
- 1 - 10 % gerad- oder verzweigtkettig Alkanole mit 2 - 5 Kohlenstoffatomen oder ihre Mischungen,
- 0,1 - 3% lipophiler Solubilisierungsagentien.

19. Zusammensetzung nach Anspruch 18, **dadurch gekennzeichnet, dass** sie in Gew.% aufweist:
0,5 - 5% eines Zusatzes nach einem der Ansprüche 7 - 9
1 - 10% Alkylpolyglycoside mit 8 - 14 Kohlenstoffatome in der Alkylkette,
- 1 - 10% gerad- oder verzweigtkettige Alkanole mit 2 - 5 Kohlenstoffatome oder ihre Mischungen, 0,1 - 2% etherisches Öl,
0 - 0,5% Konservierungsmittel.

20. Zusammensetzung nach Anspruch 19 **dadurch gekennzeichnet, dass** das etherische Öl ausgewählt ist aus Ölen aus Kiefer, Zitrone, Orange, Mandarine, Pampelmuse, Lavendel, Minze, Thymian, Rosmarin, Eucalyptus.

21. Verwendung des Zusatzes nach den Ansprüchen 9 - 14 in der Kosmetik, Hautpflege, Pharmazie, Pflanzenschutzmitteln.
